# EUROPÄISCHE PATENTSCHRIFT

(11) **EP 1 269 992 B1**
(45) Veröffentlichungstag und Bekanntmachung des Hinweises auf die Patenterteilung: **07.12.2005**
(21) Anmeldenummer: 02012371.7
(22) Anmeldetag: 06.06.2002
(51) Int. Cl.: A61K 7/50, A61K 7/06, A61K 7/48, C07C 211/63, C11D 1/62, C11D 3/20

(54) **Zusammensetzungen enthaltend quaternäre Ammoniumverbindungen**
Compositions comprising quaternary ammonium compounds
Compositions comprenant des composés d'ammonium quaternaire

(30) Priorität: 19.06.2001 DE 10129225
(43) Veröffentlichungstag der Anmeldung: 02.01.2003
(60) Teilanmeldung: 05014387.4
(73) Patentinhaber: Clariant GmbH, 65929 Frankfurt am Main (DE)
(72) Erfinder: Milbradt, Robert, Dr., 65189 Wiesbaden (DE); Klein, Sonja, 65795 Hattersheim (DE); Scherl, Franz Xaver, Dr., 84508 Burgkirchen (DE); Gatter, Erich, Dr., 84556 Kastl (DE); Oberhauser, Adelgunde, 84524 Neuötting (DE)
(74) Vertreter: Mikulecky, Klaus

(56) Entgegenhaltungen:
- EP-A1- 1 262 171
- WO-A-00/28950
- WO-A-91/12880
- DE-C- 19 714 162
- PATENT ABSTRACTS OF JAPAN vol. 011, no. 101 (C-413), 31. März 1987 (1987-03-31) & JP 61 249911 A (SANYO CHEM IND LTD), 7. November 1986 (1986-11-07)
- PATENT ABSTRACTS OF JAPAN vol. 2000, no. 05, 14. September 2000 (2000-09-14) & JP 2000 044441 A (KAO CORP), 15. Februar 2000 (2000-02-15)
- DATABASE WPI Section Ch, Week 198652 Derwent Publications Ltd., London, GB; Class A96, AN 1986-344270 XP002213736 & JP 61 260011 A (YG NONOGAWA SHODI), 18. November 1986 (1986-11-18)

## Beschreibung

Die Erfindung betrifft Zusammensetzungen, enthaltend quaternäre Ammoniumverbindungen, die einen niedrigen Stockpunkt, gute Löslichkeit bzw. Dispergierbarkeit in wässrigen Medien und einen niedrigen Flammpunkt besitzen und somit hervorragend zur Konfektionierung von quaternären Ammoniumverbindungen geeignet sind.

Kosmetische Mittel, wie z.B. Haarbehandlungsmittel, enthalten häufig in Wasser schwerlösliche quaternäre Ammoniumverbindungen, die eine langkettige Alkyl- oder Alkenylgruppe aufweisen. Solche Mittel sind üblicherweise als wässrige Dispersionen, Emulsionen, Mikroemulsionen, Gele oder auch in Aerosolform konfektioniert und werden z.B. als Shampoos, Haarkuren, Haarspülungen eingesetzt.

Für den Hersteller solcher Mittel ist es von großem Vorteil, die quaternären Ammoniumverbindungen als Compounds oder Formulierungen in Form von Flakes, Pellets oder Pasten bereitzustellen, die bei hohem kationischen Wirkstoffanteil einen niedrigen Stockpunkt sowie gute Löslichkeit bzw. Dispergierbarkeit in wässrigen Medien besitzen.
Gemäß dem Stand der Technik können die obigen Anforderungen durch die Zugabe von kurzkettigen Alkoholen, insbesondere von Isopropanol in Mengen von 15 bis 20 Gew.-%, erreicht werden. Aufgrund ihrer niedrigen Siede- und Flammpunkte sind solche kurzkettigen Alkohole jedoch problematisch.
Wie in WO 00/28950 beschrieben, können die kurzkettigen Alkohole durch lineare Fettalkohole (z.B. Cetyl-, Lauryl-, Behenyl- oder Stearylalkohol) ersetzt werden. Um den Stockpunkt bzw. Schmelzpunkt der Mischungen auf Temperaturen unterhalb 100°C herabzusetzen werden zusätzlich Glykole, wie z.B. Propylenglykol oder 1,3-Butandiol, zugesetzt.
In der WO 00/28950 wird weiterhin hervorgehoben, dass es sich bei den Fettalkoholen vorteilhafterweise um homogene Fettalkohole handelt, die weniger als ca. 10 Gew.-% eines anderen Fettalkohols enthalten.
In der WO 00/28950 wird weiterhin ausgeführt, dass der Ersatz der kurzkettigen Alkohole durch Glykole ohne gleichzeitige Zugabe von Fettalkoholen zu nicht pelletierbaren Formulierungen führt.

Überraschenderweise wurde nun gefunden, dass Zusammensetzungen, enthaltend quaternäre Ammoniumverbindungen und mindestens einen mehrwertigen Alkohol mit 5 bis 12 Kohlenstoffatomen, niedrige Stock- und Schmelzpunkte, gute Löslichkeit bzw. Dispergierbarkeit in wässrigen Medien und einen niedrigen Flammpunkt besitzen.
Überraschenderweise können die Zusammensetzungen frei sein von Fettalkoholen und linearen oder verzweigten Alkoholen mit 8 bis 36 Kohlenstoffatomen und sind dennoch bei Raumtemperatur ausreichend hart und spröde, um pelletiert oder geschuppt zu werden. Die neuartigen Zusammensetzungen eignen sich damit hervorragend zur Konfektionierung von quaternären Ammoniumverbindungen.

Gegenstand der Erfindung sind Zusammensetzungen, enthaltend, bezogen auf die fertigen zusammen setzungen
a) 60-90 Gew-% mindestens eine quaternäre Ammoniumverbindung gemäß Formel (1) 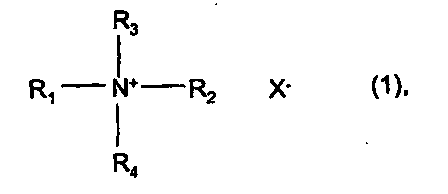 wobei
   R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen,
   und
   R₂, R₃ und R₄ unabhängig voneinander gleich oder verschieden sein können und für eine -CH₃, CH₃CH₂-,CH₃CH₂CH₂-, CH₃CH₂CH₂CH₂-, -CH₂CH₂OH oder -CH₂CH(OH)CH₂OH-Gruppe stehen
   und
   X⁻ für ein Anion steht,
   und
b) mindestens einen mehrwertigen Alkohol mit 5 bis 12 Kohlenstoffatomen darduch gekennzeichnet, dass die Zusammensetzungen frei von Fettalkoholen und linearen oder verzweigten Monoalkoholen mit 8 bis 36 Kohlenstoffatomen sind/und in Form von Pellets und Flakes vorliegen.

Der Anteil an quaternären Ammoniumverbindungen a), bezogen auf die fertigen Zusammensetzungen, beträgt bevorzugt 60 bis 90 Gew.-%, besonders bevorzugt 60 bis 85 Gew.-%, ganz besonders bevorzugt 60 bis 82 Gew.-%. Überraschenderweise zeigte sich, dass die Zusammensetzungen vorteilhafterweise hohe Gewichtsanteile an quaternären Ammoniumverbindungen a) bei gleichzeitig niedrigen Schmelz- bzw. Stockpunkten enthalten können.

Als quaternäre Ammoniumverbindungen a) bevorzugt sind (C₁₂-C₃₆)-Alkyltrimethylammoniumverbindungen, besonders bevorzugt (C₁₄-C₃₀)-Alkyltrimethylammoniumverbindungen, insbesondere bevorzugt (C₁₆-C₂₄)-Alkyltrimethylammoniumverbindungen.

Insbesondere bevorzugt sind Alkyltrimethylammoniumverbindungen bei denen der Alkylrest einen Behenyl-, Erucyl-, Cetyl- oder Stearylrest darstellt.

Beim Anion X in Formel (1) kann es sich um ein beliebiges ladungsausgleichendes Anion handeln; bevorzugt sind Chlorid, lodid, Bromid, Methosulfat, Hydrogensulfat, Lactat und/oder Citrat, besonders bevorzugt Chlorid und Methosulfat.

Ganz besonders geeignet als quaternäre Ammoniumverbindung a) ist Behenyltrimethylammoniumchlorid. Der Anteil an mehrwertigen Alkoholen b) beträgt, bezogen auf die fertigen Zusammensetzungen, bevorzugt 10 bis 70 Gew.-%, besonders bevorzugt 15 bis 60 Gew.-%, insbesondere bevorzugt 15 bis 55 Gew.-%.

Unter mehrwertigen Alkoholen b) sind solche zu verstehen, die mindestens zwei OH-Gruppen im Molekül tragen. Die mehrwertigen Alkohole b) können unverzweigt oder verzweigt und gesättigt oder ungesättigt sein. Weiterhin können die mehrwertigen Alkohole b) aus über Etherbrücken verknüpften niedermolekularen mehrwertigen Alkoholen aufgebaut sein.

Als mehrwertige Alkohole b) bevorzugt geeignet sind Pentandiol, Hexandiol, Hexyiengiykol, Trimethylpentandiol, Heptandiol, Octandiol, Nonandiol, Decandiol, Undecandiol, Dodecandiol, Diglycerin, Triglycerin, Dipropylenglykol, Tripropylenglykol, Sorbitol, Xylitol, Mannitol und/oder Mischungen derselben.

Besonders bevorzugt als mehrwertige Alkohole b) sind 1,5-Pentandiol, 1,2-Pentandiol, 1,6-Hexandiol, 1,2-Hexandiol, 1,7-Heptandiol, 1,2-Heptandiol, 1,8-Octandiol, 1,2-Octandiol, 1,9-Nonandiol, 1,2-Nonandiol, 1,10-Decandiol, 1,2-Decandiol, 1,11-Undecandiol, 1,2-Undecandiol, 1,12-Dodecandiol, 1,2-Dodecandiol, 2-Methyl-2,4-pentandiol, 2,2,4-Trimethyl-1,3-pentandiol, Diglycerin, Triglycerin, Dipropylenglykol, Tripropylenglykol, Sorbitol, Xylitol, Mannitol und/oder Mischungen derselben.

Insbesondere bevorzugt als mehrwertige Alkohole b) sind 1,6-Hexandiol, Dipropylenglykol, 2-Methyl-2,4-pentandiol, 2,2,4-Trimethyl-1,3-pentandiol und Mischungen derselben.

Besonders vorteilhafte anwendungstechnische Eigenschaften zeigen Zusammensetzungen, die mindestens einen mehrwertigen Alkohol b) mit 6 bis 8 Kohlenstoffatomen enthalten, der gegebenenfalls zusammen mit anderen mehrwertigen Alkoholen b) eingesetzt werden kann.

Optional können die erfindungsgemäßen Zusammensetzungen zur Verbesserung der anwendungstechnischen Eigenschaften unverzweigte oder verzweigte Monoalkohole mit 1 bis 4 Kohlenstoffatomen enthalten.
Bevorzugt als Monoalkohole sind Ethanol, Propanol, Isopropanol, Butanol, Isobutanol und t-Butanol, besonders bevorzugt Isopropanol.
Bevorzugt enthalten die Zusammensetzungen, bezogen auf die fertigen Zusammensetzungen, weniger als 5 Gew.-%, besonders bevorzugt weniger als 3 Gew.-%, an derartigen Monoalkoholen.
In einer bevorzugten Ausführungsform sind die Zusammensetzungen frei von unverzweigten oder verzweigten Monoalkoholen mit 1 bis 4 Kohlenstoffatomen.

Die erfindungsgemäßen Zusammensetzungen besitzen bevorzugt Stockpunkte unterhalb 100°C, besonders bevorzugt unterhalb 95°C, insbesondere bevorzugt unterhalb 90°C, ganz besonders bevorzugt unterhalb 85°C.

Die Flammpunkte der erfindungsgemäßen Zusammensetzungen liegen bevorzugt oberhalb 80°C, besonders bevorzugt oberhalb 100°C.

Bei den erfindungsgemäßen Zusammensetzungen handelt sich um Pellets, Flakes.

In einer bevorzugten Ausführungsform handelt es sich bei den erfindungsgemäßen Zusammensetzungen um Pellets.

In einer bevorzugten Ausführungsform werden die erfindungsgemäßen Zusammensetzungen gemäß Anspruch 1 dadurch hergestellt, dass eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) mindestens einen mehrwertigen Alkohol b) mit 5 bis 12 Kohlenstoffatomen und
iii) gegebenenfalls mindestens einen unverzweigten oder verzweigten Monoalkohol mit 1 bis 4 Kohlenstoffatomen.

In einer bevorzugten Ausführungsform werden die Komponenten i) bis iii) vermischt und anschließend, gegebenenfalls unter Rühren, erwärmt. Dabei wird die Temperatur so gewählt, dass eine Schmelze vorliegt. Bevorzugt sind Temperaturen von 70 bis 120 °C, besonders bevorzugt 80 bis 110°C. In einer anderen bevorzugten Ausführungsform wird die Komponente i) als Schmelze vorgelegt.
Die quaternären Ammoniumverbindungen a) der Komponente i) können in bekannter Weise durch Alkylierung eines tertiären Amins in Gegenwart mindestens eines unverzweigten oder verzweigten Monoalkohols mit 1 bis 4 Kohlenstoffatomen, bevorzugt Ethanol, Propanol, Isopropanol, Butanol, Isobutanol und t-Butanol, besonders bevorzugt Isopropanol, hergestellt werden. Die quaternären Ammoniumverbindungen werden bevorzugt als Pellets oder besonders bevorzugt als Pulver eingesetzt. In einer bevorzugten Ausführungsform enthalten die quaternären Ammoniumverbindungen weniger als 5 Gew.-%, bevorzugt weniger als 3 Gew.-%, an Monoalkoholen. In einer weiteren bevorzugten Ausführungsform enthalten die quaternären Ammoniumverbindungen 10 bis 25 Gew.-% an Monoalkoholen.
Die erfindungsgemäßen Zusammensetzungen besitzen bevorzugt einen Gesamtgehalt an unverzweigten oder verzweigten Monoalkoholen mit 1 bis 4 Kohlenstoffatomen von weniger als 5 Gew.-%, besonders bevorzugt weniger als 3 Gew.-%.
In einer ebenfalls bevorzugten Ausführungsform sind die erfindungsgemäßen Zusammensetzungen frei von unverzweigten oder verzweigten Monoalkoholen mit 1 bis 4 Kohlenstoffatomen.
Zur Einstellung des gewünschten Gehalts an Monoalkoholen in den erfindungsgemäßen Zusammensetzungen werden die Komponenten i) und/oder iii) entsprechend gewählt bzw. bemessen und/oder man entfernt die Monoalkohole teilweise oder ganz vorab aus der Komponente i).
In einer weiteren Ausführungsform werden die Monoalkohole nachträglich aus den erfindungsgemäßen Zusammensetzungen bis auf den gewünschten Restgehalt entfernt. Bevorzugt werden die Monoalkohole bei 700 bis 10 mbar, bevorzugt 400 bis 70 mbar, und 60 bis 90°C abgezogen.
Ebenso können die Monoalkohole bei Normaldruck in geeigneten Verdampfungseinrichtungen (z.B. Dünnschicht-Verdampfer) bei Temperaturen bis 120 °C abdestilliert werden.

Überraschenderweise wurde gefunden, dass die erfindungsgemäßen Zusammensetrungen gemäß Anspruch 1 auch "in situ" durch Alkylierung von
i) mindestens einem tertiären Amin NR₁R₂R₃,
   wobei R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen,
   und R₂ und R₃ unabhängig voneinander gleich oder verschieden sein können und für CH₃-, CH₃CH₂-, CH₃CH₂CH₂-, CH₃CH₂CH₂CH₂- oder -CH₂CH₂(OH) stehen, durch
ii) mindestens ein Alkylierungsmittel, ausgewählt aus
   a) R₄X, wobei R₄ für -CH₃, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
   b) Ethylenoxid und einer Säure HX, wobei X für Cl, I, Br, OSO₃H, Citrat oder Lactat steht,
   in Gegenwart von
   iii) mindestens einem mehrwertigen Alkohol b) mit 5 bis 12 Kohlenstoffatomen und
   iv) gegebenenfalls mindestens einem unverzweigten oder verzweigten Monoalkohol mit 1 bis 4 Kohlenstoffatomen
   hergestellt werden können.

Bevorzugt werden bei der Umsetzung die Einsatzmengen an mehrwertigen Alkoholen b) und gegebenenfalls unverzweigten oder verzweigten Monoalkoholen mit 1 bis 4 Kohlenstoffatomen entsprechend den schon vorab beschriebenen bevorzugten Zusammensetzungen der erfindungsgemäßen Zusammensetzungen gewählt. Die Gehalte können auch durch nachträgliche Zugabe oder Entfernen der Komponenten eingestellt werden.

In einer bevorzugten Ausführungsform werden bei der Umsetzung keine unverzweigten oder verzweigten Monoalkohole mit 1 bis 4 Kohlenstoffatomen eingesetzt.

Die nach den oben beschriebenen Verfahren hergestellten erfindungsgemäßen Zusammensetzungen werden als homogene oder inhomogene Schmelze durch Abkühlen inPellets oder Flakes, überführt.

Bevorzugt werden die Zusammensetzungen in Pellets weiterverarbeitet.

Die erfindungsgemäßen Zusammensetzungen eignen sich allgemein zur Herstellung von Mitteln, enthaltend quaternäre Ammoniumverbindungen.
Besonders geeignet sind die Zusammensetzungen zur Herstellung von kosmetischen, dermatologischen und pharmazeutischen Mitteln.
Insbesondere eignen sich die Zusammensetzungen zur Herstellung von Haarbehandlungsmitteln.

Gegenstand der Erfindung ist demnach auch die Verwendung der erfindungsgemäßen Zusammensetzungen zur Herstellung von Mitteln, bevorzugt kosmetischen, dermatologischen und pharmazeutischen Mitteln, insbesondere Haarbehandlungsmitteln, enthaltend quaternäre Ammoniumverbindungen.

Beispiele für bevorzugte Mittel sind Shampoos, rinse-off-Haarconditionierer, Cremespülungen, Klarspülungen, Haarkuren, Haarfärbe- und Haartönungsmittel, Dauerwellmittel, Haargele, Haarconditionierer in Aerosol-, Spray- und Fluidform, 2-in-1 Duschbäder, Cremeduschbäder, Hautpflegemittel, Tagescremes, Nachtcremes, Pflegecremes, Nährcremes, Bodylotions und Salben.

Die kosmetischen, dermatologischen und pharmazeutischen Mittel enthalten die erfindungsgemäßen Zusammensetzungen, bezogen auf die fertigen Mittel, bevorzugt in Mengen von 0,1 bis 15 Gew.-%, besonders bevorzugt 1 bis 10 Gew.-%, insbesondere bevorzugt 1 bis 7 Gew.-%.

Die kosmetischen, dermatologischen und pharmazeutischen Mittel können als weitere Hilfs- und Zusatzstoffe alle üblichen Tenside, Ölkörper, Emulgatoren und Co-Emulgatoren, kationischen Polymere, Filmbildner, Überfettungsmittel, feuchtigkeitsspendende Mittel, Stabilisatoren, biogene Wirkstoffe, Konservierungsmittel, Perlglanzmittel, Farb- und Duftstoffe, Lösungsmittel, Glycerin, Hydrotrope, Trübungsmittel, Verdickungsmittel, Dispergiermittel, Eiweißderivate, wie z.B. Gelatine, Collagenhydrolysate, Polypeptide auf natürlicher und synthetischer Basis, Eigelb, Lecithin, Lanolin und Lanolinderivate, Silicone, deodorierende Mittel, Stoffe mit keratolytischer und keratoplastischer Wirkung, Enzyme, Trägersubstanzen, Antioxidantien, UV-Lichtschutzfilter, Pigmente und Metalloxide, sowie antimikrobiell wirkende Agentien enthalten.

Als Tenside können anionische, kationische, nichtionische, amphotere und/oder zwitterionische Tenside verwendet werden.
Bevorzugte nichtionische Tenside enthalten als hydrophile Gruppe eine Polyolgruppe, eine Polyolalkenylethergruppe oder eine Kombination aus Polyol- und Polyglykolethergruppen. Bevorzugt sind Anlagerungsprodukte aus von 2 bis 30 Mol Ethylenoxid, 2 bis 30 Mol Ethylenoxid zusammen mit bis 5 Mol Propylenoxid oder von bis 5 Mol Propylenoxid an lineare Fettalkohole mit 8 bis 22 C-Atomen und Alkylphenolen mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und -diester von gesättigten und ungesättigten (C₈C₁₈)-Fettsäuren und deren Ethylenoxidanlagerungsprodukte, (C₈-C₁₈)-Alkylmono- und -oligoglykoside und deren ethoxylierte Analoga, Anlagerungsprodukte von 10 bis 60 Mol Ethylenoxid an Ricinusöl und gehärtetes Ricinusöl, ethoxylierte und nicht ethoxylierte Mono-, Di- und Trialkylmonophosphorsäureester, insbesondere Mono-, Di- und Tri-(Lauryltetraglycolether)-o-Phosphor-säureester und Mono-, Di- und Tri-(Cetyltetraglycolether)-o-Phosphorsäureester.

Bevorzugte amphotere Tenside tragen eine (C₈-C₁₈)-Alkyl- oder -Acylgruppe und mindestens eine freie Aminogruppe und mindestens eine -COOH- oder -SO₃H-Gruppe. Bevorzugt sind N-Acylglycine, N-Alkylpropionsäure, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe. Besonders bevorzugte sind N-Kokosalkylaminopropionat, Kokosacylaminoethylaminopropionat und (C₁₂-C₁₈)-Alkylsarcosine.

Besonders geeignete zwitterionische Tenside sind Betaine, wie beispielsweise die N-Alkyl-N,N-dimethylammoniumglycinate, z.B. Kokosalkyldimethylammoniumglycinate, N-Acyl-aminopropyl-N-N-dimethylammoniumglycinate, z.B. Kokosacylaminopropyl-dimethylammoniumglycinat, 2-Alkyl-3-carboxymethyl-3-hydroxyethylimidazoline mit jeweils 8 bis 18 C-Atomen in der Alkyl- oder Acylgruppe und das Kokosacylaminoethylhydroxyethylcarboxymethylglycinat.

Bevorzugt enthalten die Mittel Tensid-Mischungen, besonders bevorzugt sind Mischungen aus nichtionischen und zwitterionischen oder amphoteren Tensiden in einem Gewichtsverhältnis von 5 zu 1 bis 1 zu 5 oder Mischungen aus nichtionogenen Tensiden und beliebigen Mischungen aus zwitterionischen und amphoteren Tensiden in einem Gewichtsverhältnis von 5 zu 1 bis 1 zu 5.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12 bis 24 C-Atomen, Fettsäureester mit insgesamt 12 bis 26 C-Atomen, flüssige Kohlenwasserstoffe mit 10 bis 32 C-Atomen und Gemische davon. Geeignete Fettsäureester sind z.B. Methylpalmitat, Ethyloleat, Isopropylmyristat, n-Hexyllaurat, n-Butylstearat und Cetyl-/ Stearylisononanoat. Besonders bevorzugt sind Paraffinöle, Vaseline, Pflanzenöle, synthetische Triglyceride wie z.B. Glyceryltricaprylat, sowie Silikonöle.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. Als Konsistenzgeber kommen Fettalkohole mit 12 bis 22, vorzugsweise 12 bis 18 Kohlenstoffatomen sowie Partialglyceride in Betracht.

Als weitere Verdickungsmittel können Polysaccharide, insbesondere Xantham-Gum, Guar-Guar, Agar-Agar, Alginate, Carboxymethylcellulose, Hydroxyethylcellulose, höhermolekulare Polyethylenglykolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylpropan, Fettalkoholethoxylate oder Alkyloligoglucoside, sowie Elektrolyte wie Kochsalz und Ammoniumchlorid eingesetzt werden.

Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/ oder alkylmodifizierte Siliconverbindungen, die bei Raumtemperatur sowohl flüssig als auch harzförmig vorliegen können.

Unter biogenen Wirkstoffen sind beispielsweise Bisabolol®, Allantoin®, Phytantriol®, Panthenol®, AHA-Säuren, Pflanzenextrakte und Vitaminkomplexe zu verstehen.

Als Antischuppenmittel können Climbazol®, Octopirox®, Oxiconazol® und Zinkpyrethion® eingesetzt werden.

Gebräuchliche Filmbildner sind Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner Hydrotrope wie beispielsweise Ethanol, Isopropylalkohol, Propylenglykol oder Glucose eingesetzt werden.

Als Konservierungsmittel eignen sich beispielsweise Phenoxyethanol, Formaldehydlösung, Parabene, Pentandibl und Sorbinsäure.

Als feuchtigkeitsspendende Substanz stehen beispielsweise Isopropylpalmitat, Glycerin und/oder Sorbitol zu Verfügung.

Der Gesamtanteil an Hilfs- und Zusatzstoffen in den Mitteln beträgt bevorzugt 1 bis 50 Gew.-%, besonders bevorzugt 5 bis 40 Gew.-%.

Die nachfolgenden Beispiele dienen der Erläuterung der Erfindung, ohne sie jedoch einzuschränken. Der Quat-Aktivgehalt der erfindungsgemäßen Zusammensetzungen wurde durch Kation-Titration bestimmt. Die Stockpunkte wurden durch langsames Absenken der Temperatur ermittelt.

### Beispiel 1:

110,54 g ®Genamin KDMP (Behenyltrimethylammoniumchlorid Pellets mit einem Gehalt von ca. 19 % Isopropanol, Fa. Clariant GmbH) wurden in 38,37 g Dipropylenglykol in einem 1000 ml-Rundkolben gemischt und in einem Ölbad bei 70 - 90°C aufgeschmolzen. Nachdem sich eine homogene Schmelze gebildet hatte wurde am Rotationsverdampfer ein Vakuum von zunächst ca. 700 mbar angelegt und das Isopropanol während etwa 3 Stunden abdestilliert. Dabei wurde das Vakuum kontinuierlich auf etwa 22 mbar erhöht. Anschließend entfernte man Reste des flüchtigen Lösemittels während 2 Stunden bei 90°C Badtemperatur und 16 mbar Vakuum. Man erhielt eine klare Lösung, die bei 69 - 73°C erstarrte und bei 90°C wieder vollständig geschmolzen vorlag. Bei 25°C war die Zusammensetzung wachsartig fest. Der Quat-Aktivgehalt betrug 70 Gew.-%.

### Beispiel 2:

Analog Beispiel 1 wurde eine Zusammensetzung aus
75 Gew.-% Behenyltrimethylammoniumchlorid, 22 Gew.-% Dipropylenglykol und
3 Gew.-% Isopropanol hergestellt. Man erhielt eine klare Lösung, die bei ca. 73-77°C erstarrte und bei 90°C wieder vollständig geschmolzen vorlag. Bei 25°C war die Zusammensetzung wachsartig fest. Der Quat-Aktivgehalt betrug 75 Gew.-%.

### Beispiel 3:

Analog Beispiel 1 wurde eine Zusammensetzung aus
75 Gew.-% Behenyltrimethylammoniumchlorid, 23,7 Gew.-% 1,6-Hexandiol und
1,3 Gew.-% Isopropanol hergestellt. Man erhielt eine klare Lösung, die bei ca. 71°C erstarrte und bei 90°C wieder vollständig geschmolzen vorlag. Bei 25°C war die Zusammensetzung wachsartig fest. Der Quat-Aktivgehalt betrug 75 Gew.-%.

Formulierungsbeispiele für kosmetische Formulierungen:
Die erfindungsgemäße Zusammensetzung aus Beispiel 3 wurde aufgeschmolzen und durch Auftropfen auf eine kalte Metallplatte pelletiert. Die so erhaltenen Pellets wurden dann in die entsprechenden Formulierungen eingearbeitet.

### Beispiel 4: Cremespülung

| | | |
|---|---|---|
| A | Zusammensetzung aus Bsp. 3 | 2 Gew.-% |
| | ®HOSTAPHAT KL 340 D | 1,5 Gew.-% |
| | (Trilaureth-4 Phosphate, Fa. Clariant) | |
| | Cetylalkohol | 3 Gew.-% |
| | Paraffinöl | 1 Gew.-% |
| | | |
| B | Wasser | 92,5 Gew.-% |
| | | |
| C | Citronensäure | q.s. |

### Herstellung:

I) A bei 75°C aufschmelzen
II) B auf 75°C erwärmen
III) B in A einrühren und Abkühlen
IV) pH-Wert mit C auf pH = 4 einstellen

### Beispiel 5: O/W-Handcreme

| | | |
|---|---|---|
| A | Zusammensetzung aus Bsp. 3 | 2,7 Gew.-% |
| | ®HOSTACERIN DGSB | 6 Gew.-% |
| | (PEG-4 Polyglyceryl-2 Stearate, Fa. Clariant) | |
| | Paraffinöl, hochviskos | 10 Gew.-% |
| | Isopropylpalmitat | 10 Gew.-% |
| | | |
| B | Wasser | 70,9 Gew.-% |
| | Konservierungsmittel | q.s. |
| | | |
| C | Parfüm | 0,4 Gew.-% |

### Herstellung:

I) A bei 80°C aufschmelzen
II) B auf 80°C erwärmen
III) B in A einrühren und Abkühlen
IV) C bei 35°C zugeben

### Beispiel 6: Haarconditionierer mit Perlglanzeffekt

| | | |
|---|---|---|
| A | Zusammensetzung aus Bsp. 3 | 2 Gew.-% |
| | ®Genamin KSL | 9 Gew.-% |
| | (PEG- 5 Stearyl Ammonium Lactate, Fa. Clariant) | |
| | ®Hostaphat KL 340 D | 1,5 Gew.-% |
| | (Trilaureth- 4 Phosphate, Fa. Clariant) | |
| | Jojobaöl | 1,0 Gew.-% |
| | | |
| B | ®Tylose H 100 000 YP2 | 1,5 Gew.-% |
| | (Hydroxyethylcellulose, Fa. Clariant) | |
| | | |
| C | Wasser | ad 100 % |
| | | |
| D | Parfüm | 0,50 % |
| | Panthenol | 0,50 % |
| | ®Genapol PDC | 4 Gew.-% |
| | (Glycol Distearate, Laureth-4, Cocamidopropyl | |
| | Betaine, Glimmer und Titandioxid, Fa. Clariant) | |
| | | |
| E | Citronensäure | q.s. |

### Herstellung:

I) A wird auf 75°C erwärmt
II) B wird sorgfältig in C gelöst und auf etwa 75°C erwärmt
III) II wird unter Rühren zu I gegeben
IV) Unter Rühren abkühlen lassen, bei 30°C D in III geben
V) pH-Wert mit E auf pH=4 einstellen

## Patentansprüche

1. Zusammensetzungen, enthaltend, bezogen auf die fertigen Zusammensetzungen,
a) 60 bis 90 Gew.-% mindestens einer quaternären Ammoniumverbindung gemäß Formel (1) 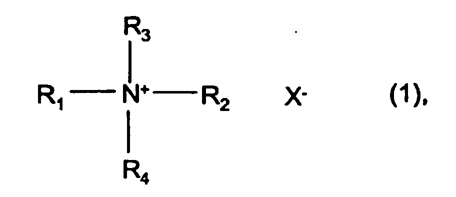 wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen,
und
R₂, R₃ und R₄ unabhängig voneinander gleich oder verschieden sein können und für eine -CH₃, CH₃CH₂-,CH₃CH₂CH₂-, CH₃CH₂CH₂CH₂-, CH₂CH₂OH oder -CH₂CH(OH)CH₂OH-Gruppe stehen
und
X⁻ für ein Anion steht,
und
b) mindestens einen mehrwertigen Alkohol mit 5 bis 12 Kohlenstoffatomen, **dadurch gekennzeichnet, dass** sie frei sind von Fettalkoholen und unverzweigten oder verzweigten Monoalkoholen mit 8 bis 36 Kohlenstoffatomen und in Form von Pellets oder Flakes vorliegen.

2. Zusammensetzungen nach Anspruch 1, **dadurch gekennzeichnet, dass** der Gehalt an quaternären Ammoniumverbindungen a), bezogen auf die fertigen Zusammensetzungen, 60 bis 85 Gew.% beträgt.

3. Zusammensetzungen nach Anspruche 1 oder 2, **dadurch gekennzeichnet, dass** es sich bei den quaternären Ammoniumverbindungen a) um (C₁₂-C₃₆)-, bevorzugt (C₁₄-C₃₀)-, besonders bevorzugt (C₁₆-C₂₄)-Alkyltrimethylammoniumverbindungen, handelt.

4. Zusammensetzungen nach mindestens einem der Ansprüche 1 bis 3, **dadurch gekennzeichnet, dass** es sich beim Anion X⁻ in Formel (1) um Chlorid, lodid, Bromid, Methosulfat, Hydrogensulfat, Lactat und/oder Citrat, bevorzugt Chlorid und/oder Methosulfat, handelt.

5. Zusammensetzungen nach mindestens einem der Ansprüche 1 bis 4, **dadurch gekennzeichnet, dass** es sich bei der quaternären Ammoniumverbindung a) um Behenyltrimethylammoniumchlorid handelt.

6. Zusammensetzungen nach mindestens einem der Ansprüche 1 bis 5, **dadurch gekennzeichnet, dass** der Gehalt an mehrwertigen Alkoholen b), bezogen auf die fertigen Zusammensetzungen, 10 bis 70 Gew.-%, bevorzugt 15 bis 60 Gew.-%, besonders bevorzugt 15 bis 55 Gew.-%, beträgt.

7. Zusammensetzungen nach mindestens einem der Ansprüche 1 bis 6, **dadurch gekennzeichnet, dass** es sich bei den mehrwertigen Alkoholen b) um Pentandiol, Hexandiol, Hexylenglykol, Trimethylpentandiol, Heptandiol, Octandiol, Nonandiol, Decandiol, Undecandiol, Dodecandiol, Diglycerin, Triglycerin, Dipropylenglykol, Tripropylenglykol, Sorbitol, Xylitol, Mannitol und/oder Mischungen derselben handelt.

8. Zusammensetzungen nach Anspruch 7, **dadurch gekennzeichnet, dass** es sich bei den mehrwertigen Alkoholen b) um 1,5-Pentandiol, 1,2-Pentandiol, 1,6-Hexandiol, 1,2-Hexandiol, 1,7-Heptandiol, 1,2-Heptandiol, 1,8-Octandiol, 1,2-Octandiol, 1,9-Nonandiol, 1,2-Nonandiol, 1,10-Decandiol, 1,2-Decandiol, 1,11-Undecandiol, 1,2-Undecandiol, 1,12-Dodecandiol, 1,2-Dodecandiol, 2-Methyl-2,4-pentandiol, 2,2,4-Trimethyl-1,3-pentandiol, Diglycerin, Triglycerin, Dipropylenglykol, Tripropylenglykol, Sorbitol, Xylitol, Mannitol und/oder Mischungen derselben handelt.

9. Zusammensetzungen nach Anspruch 8, **dadurch gekennzeichnet, dass** es sich bei den mehrwertigen Alkohole b) um 1,6-Hexandiol, 2-Methyl-2,4-pentandiol, 2,2,4-Trimethyl-1,3-pentandiol und/oder Dipropylenglykol handelt.

10. Zusammensetzungen nach mindestens einem der Ansprüche 1 bis 8, **dadurch gekennzeichnet, dass** sie mindestens einen mehrwertigen Alkohol b) mit 6 bis 8 Kohlenstoffatomen enthalten.

11. Zusammensetzungen nach mindestens einem der Ansprüche 1 bis 10, **dadurch gekennzeichnet, dass** sie, bezogen auf die fertigen Zusammensetzungen, weniger als 5 Gew.-%, bevorzugt weniger als 3 Gew.-%, an unverzweigten oder verzweigten Monoalkoholen mit 1 bis 4 Kohlenstoffatomen enthalten.

12. Zusammensetzungen nach Anspruch 11, **dadurch gekennzeichnet, dass** es sich bei den Monoalkoholen um Ethanol, Propanol, Isopropanol, Butanol, Isobutanol und t-Butanol, bevorzugt Isopropanol, handelt.

13. Zusammensetzungen nach mindestens einem der Ansprüche 1 bis 12, **dadurch gekennzeichnet, dass** sie Stockpunkte unterhalb 100°C, bevorzugt unterhalb 95°C, besonders bevorzugt unterhalb 90°C, insbesondere bevorzugt unterhalb 85 °C, besitzen.

14. Zusammensetzungen nach mindestens einem der Ansprüche 1 bis 13, **dadurch gekennzeichnet, dass** sie Flammpunkte oberhalb 80°C, bevorzugt oberhalb 100°C, besitzen.

15. Zusammensetzungen nach mindestens einem der Ansprüche 1 bis 14, **dadurch gekennzeichnet, dass** die Zusammensetzungen in Form von Pellets vorliegen.

16. Verfahren zur Herstellung von Zusammensetzungen gemäß mindestens einem der Ansprüche 1 bis 15, **dadurch gekennzeichnet, dass** eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) mindestens einen mehrwertigen Alkohol mit 5 bis 12 Kohlenstoffatomen und
iii) gegebenenfalls mindestens einen unverzweigten oder verzweigten Monoalkohol mit 1 bis 4 Kohlenstoffatomen.

17. Verfahren zur Herstellung einer Zusammensetzung gemäß mindestens einem der Ansprüche 1 bis 15, **dadurch gekennzeichnet, dass**
i) mindestens ein tertiäres Amin NR₁R₂R₃, wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen,
und R₂ und R₃ unabhängig voneinander gleich oder verschieden sein können und für -CH₃, CH₃CH₂-, CH₃CH₂CH₂-, CH₃CH₂CH₂CH₂- oder -CH₂CH₂(OH) stehen,
durch
ii) mindestens ein Alkylierungsmittel, ausgewählt aus
a) R₄X, wobei R₄für-CH₃-, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
b) Ethylenoxid und einer Säure HX, wobei X für Cl, I, Br, OSO₃H, Citrat oder Lactat steht,
in Gegenwart von
iii) mindestens einem mehrwertigen Alkohol mit 5 bis 12 Kohlenstoffatomen und
iv) gegebenenfalls mindestens einem unverzweigten oder verzweigten Monoalkohol mit 1 bis 4 Kohlenstoffatomen
alkyliert wird.

18. Verwendung von Zusammensetzungen gemäß mindestens einem der Ansprüche 1 bis 15 zur Herstellung von kosmetischen, dermatologischen und pharmazeutischen Mitteln, bevorzugt Haarbehandlungsmitteln.

19. Verwendung nach Anspruch 18, **dadurch gekennzeichnet, dass** es sich bei den Haarbehandlungsmitteln um Shampoos, rinse-off-Haarconditioner, Cremespülungen, Klarspülungen, Haarkuren, Haarfärbe- und Haartönungsmittel, Dauerwellmittel, Haargele oder Haarconditioner in Aerosol-, Spray- und Fluidform handelt.

## Claims

1. A composition comprising, based on the finished composition,
a) 60 to 90% by weight of at least one quaternary ammonium compound according to formula (1) 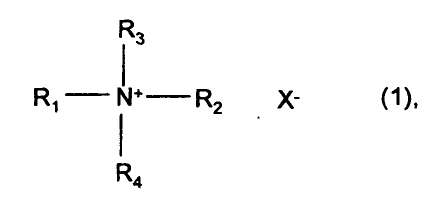 where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8,
and
R₂, R₃ and R₄, independently of one another, may be identical or different and are a -CH₃, CH₃CH₂-, CH₃CH₂CH₂-, CH₃CH₂CH₂CH₂-, CH₂CH₂OH or -CH₂CH(OH)CH₂OH
group
and
X⁻ is an anion,
and
b) at least one polyhydric alcohol having 5 to 12 carbon atoms, which is free from fatty alcohols and linear or branched monoalcohols having 8 to 36 carbon atoms and is in the form of pellets or flakes.

2. The composition as claimed in claim 1, wherein the content of quaternary ammonium compounds a), based on the finished composition, is 60 to 85% by weight

3. The composition as claimed in claim 1 or 2,
wherein the quaternary ammonium compounds a) are (C₁₂-C₃₆)-, preferably (C₁₄-C₃₀)-, particularly preferably (C₁₆-C₂₄)-alkyltrimethylammonium compounds.

4. The composition as claimed in at least one of claims 1 to 3, wherein the anion X⁻ in formula (1) is chloride, iodide, bromide, methosulfate, hydrogensulfate, lactate and/or citrate, preferably chloride and/or methosulfate.

5. The composition as claimed in at least one of claims 1 to 4, wherein the quaternary ammonium compound a).is behenyltrimethylammonium chloride.

6. The composition as claimed in at least one of claims 1 to 5, wherein the content of polyhydric alcohols b), based on the finished composition, is 10 to 70% by weight, preferably 15 to 60% by weight, particularly preferably 15 to 55% by weight.

7. The composition as claimed in at least one of claims 1 to 6, wherein the polyhydric alcohols b) are pentanediol, hexanediol, hexylene glycol, trimethylpentanediol, heptanediol, octanediol, nonanediol, decanediol, undecanediol, dodecanediol, diglycerol, triglycerol, dipropylene glycol, tripropylene glycol, sorbitol, xylitol, mannitol and/or mixtures thereof.

8. The composition as claimed in claim 7, wherein the polyhydric alcohols b) are 1,5-pentanediol, 1,2-pentanediol, 1,6-hexanediol, 1,2-hexanediol, 1,7-heptanediol, 1,2-heptanediol, 1,8-octanediol, 1,2-octanediol, 1,9-nonanediol, 1,2-nonanediol, 1,10-decanediol, 1,2-decanediol, 1,11-undecanediol, 1,2-undecanediol, 1,12-dodecanediol, 1,2-dodecanediol, 2-methyl-2,4-pentanediol, 2,2,4-trimethyl-1,3-pentanediol, diglycerol, triglycerol, dipropylene glycol, tripropylene glycol, sorbitol, xylitol, mannitol and/or mixtures thereof.

9. The composition as claimed in claim 8, wherein the polyhydric alcohols b) are 1,6-hexanediol, 2-methyl-2,4-pentanediol, 2,2,4-trimethyl-1,3-pentanediol and/or dipropylene glycol.

10. The composition as claimed in at least one of claims 1 to 8, which comprises at least one polyhydric alcohol b) having 6 to 8 carbon atoms.

11. The composition as claimed in at least one of claims 1 to 10, which comprises, based on the finished composition, less than 5% by weight, preferably less than 3% by weight, of unbranched or branched monoalcohols having 1 to 4 carbon atoms.

12. The composition as claimed in claim 11, wherein the monoalcohols are ethanol, propanol, isopropanol, butanol, isobutanol and tert-butanol, preferably isopropanol.

13. The composition as claimed in at least one of claims 1 to 12, which has a setting point below 100°C, preferably below 95°C, particularly preferably below 90°C, in particular below 85°C.

14. The composition as claimed in at least one of claims 1 to 13, which has a flash point above 80°C, preferably above 100°C.

15. The composition as claimed in at least one of claims 1 to 14, which is in the form of pellets.

16. A process for the preparation of compositions as claimed in at least one of claims 1 to 15, which comprises preparing a mixture comprising
i) at least one quaternary ammonium compound a), optionally containing a branched or unbranched monoalcohol having 1 to 4 carbon atoms,
ii) at least one polyhydric alcohol having 5 to 12 carbon atoms and
iii) optionally at least one unbranched or branched monoalcohol having 1 to 4 carbon atoms.

17. A process for the preparation of a composition as claimed in at least one of claims 1 to 15, which comprises alkylating
i) at least one tertiary amine NR₁R₂R₃, where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8, and R₂ and R₃, independently of one another, may be identical or different and are -CH₃, CH₃CH₂-, CH₃CH₂CH₂-, CH₃CH₂CH₂CH₂- or -CH₂CH₂(OH),
by
ii) at least one alkylating agent chosen from
a) R₄X, where R₄ is -CH₃-, CH₃CH₂-, CH₃CH₂CH₂-, CH₃CH₂CH₂CH₂- or
-CH₂CH (OH) CH₂ (OH), and X is Cl, I, Br, OSO₃H or methosulfate, and/or
b) ethylene oxide and an acid HX, where X is Cl, I, Br, OSO₃H, citrate or lactate,
in the presence of
iii) at least one polyhydric alcohol having 5 to 12 carbon atoms and
iv) optionally at least one unbranched or branched monoalcohol having 1 to 4 carbon atoms.

18. The use of compositions as claimed in at least one of claims 1 to 15 for the preparation of cosmetic, dermatological and pharmaceutical compositions, preferably hair-treatment compositions.

19. The use as claimed in claim 18, wherein the hair-treatment compositions are shampoos, rinse-off hair conditioners, cream rinses, clear rinses, hair cures, hair colorants and hair tints, permanent waving compositions, hair gels and hair conditioners in aerosol form, spray form and fluid form.

## Revendications

1. Compositions comprenant, par rapport aux compositions finies,
a) 60% à 90% en poids d'au moins un composé d'ammonium quaternaire de formule (1) 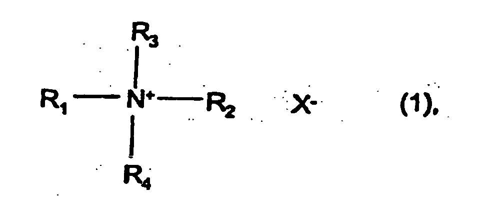 dans laquelle
R₁ représente un groupe alkyle ou alcényle non ramifié ou ramifié contenant 12 à 36 atomes de carbone, un groupe R₅CONH(CH₂)ₙ- ou un groupe R₅COO(CH₂)ₙ-, dans lesquels R₅ représente un groupe alkyle ou alcényle contenant 12 à 36 atomes de carbone et n représente un nombre allant de 1 à 8,
et
R₂, R₃ et R₄ peuvent être, indépendamment les uns des autres, identiques ou différents et représentent un groupe -CH₃, CH₃CH₂-, CH₃CH₂CH₂-, CH₃CH₂CH₂CH₂-, CH₂CH₂OH ou -CH₂CH(OH)CH₂OH-
et
X- représente un anion
et
b) au moins un polyol contenant 5 à 12 atomes de carbone,
**caractérisées en ce qu'**elles sont exemptes d'alcools gras et de monoalcools non ramifiés ou ramifiés contenant 8 à 36 atomes de carbone et **en ce qu'**elles se présentent sous forme de granulés ou de flocons.

2. Compositions selon la revendication 1, **caractérisées en ce que** la teneur en composés d'ammonium quaternaire a), par rapport aux compositions finies, va de 60% à 85% en poids.

3. Compositions selon la revendication 1 ou 2, **caractérisées en ce que** les composés d'ammonium quaternaire a) sont des composés alkyl(en C₁₂ à C₃₆) triméthylammonium, de préférence alkyl (en C₁₄ à C₃₀) triméthylammonium, de manière plus particulièrement préférée alkyl (en C₁₆ à C₂₄) triméthylammonium.

4. Compositions selon au moins l'une des revendications 1 à 3, **caractérisées en ce que** l'anion X⁻ dans la formule (1) est un anion chlorure, iodure, bromure, méthosulfate, hydrogénosulfate, lactate et/ou citrate, de préférence chlorure et/ou méthosulfate.

5. Compositions selon au moins l'une des revendications 1 à 4, **caractérisées en ce que** le composé d'ammonium quaternaire a) est le chlorure de béhényltriméthylammonium.

6. Compositions selon au moins l'une des revendications 1 à 5, **caractérisées en ce que** la teneur en polyols b), par rapport aux compositions finies, va de 10% à 70% en poids, de préférence de 15% à 60% en poids et de manière plus particulièrement préférée de 15% à 55% en poids.

7. Compositions selon au moins l'une des revendications 1 à 6, **caractérisées en ce que** les polyols b) sont le pentanediol, l'hexanediol, l'hexylèneglycol, le triméthylpentanediol, l'heptanediol, l'octanediol, le nonanediol, le décanediol, l'undécanediol, le dodécanediol, la diglycérine, la triglycérine, le dipropylèneglycol, le tripropylèneglycol, le sorbitol, le xylitol, le mannitol et/ou des mélanges de ceux-ci.

8. Compositions selon la revendication 7, **caractérisées en ce que** les polyols b) sont le 1,5-pentanediol, le 1,2-pentanediol, le 1,6-hexanediol, le 1,2-hexanediol, le 1,7-heptanediol, le 1,2-heptanediol, le 1,8-octanediol, le 1,2-octanediol, le 1,9-nonanediol, le 1,2-nonanediol, le 1,10-décanediol, le 1,2-décanediol, le 1,11-undécanediol, le 1,2-undécanediol, le 1,12-dodécanediol, le 1,2-dodécanediol, le 2-méthyl-2,4-pentanediol, le 2,2,4-triméthyl-1,3-pentanediol, la diglycérine, la triglycérine, le dipropylèneglycol, le tripropylèneglycol, le sorbitol, le xylitol, le mannitol et/ou des mélanges de ceux-ci.

9. Compositions selon la revendication 8, **caractérisées en ce que** les polyols b) sont le 1,6-hexanediol, le 2-méthyl-2,4-pentanediol, le 2,2,4-triméthyl-1,3-pentanediol et/ou le dipropylèneglycol.

10. Compositions selon au moins l'une des revendications 1 à 8, **caractérisées en ce qu'**elles contiennent au moins un polyol b) contenant 6 à 8 atomes de carbone.

11. Compositions selon au moins l'une des revendications 1 à 10, **caractérisées en ce qu'**elles contiennent, par rapport aux compositions finies, moins de 5% en poids, de préférence moins de 3% en poids de monoalcools non ramifiés ou ramifiés contenant 1 à 4 atomes de carbone.

12. Compositions selon la revendication 11, **caractérisées en ce que** les monoalcools sont l'éthanol, le propanol, l'isopropanol, le butanol, l'isobutanol et le t-butanol, de préférence l'isopropanol.

13. Compositions selon au moins l'une des revendications 1 à 12, **caractérisées en ce que** leur point d'écoulement se situe en dessous de 100°C, de préférence en dessous de 95°C, de manière plus particulièrement préférée en dessous de 90°C et de manière tout particulièrement préférée en dessous de 85°C.

14. Compositions selon au moins l'une des revendications 1 à 13, **caractérisées en ce que** leur point d'inflammation se situe au-dessus de 80°C, de préférence au-dessus de 100°C.

15. Compositions selon au moins l'une des revendications 1 à 14, **caractérisées en ce que** les compositions se présentent sous forme de granulés.

16. Procédé de préparation de compositions selon au moins l'une des revendications 1 à 15, **caractérisé en ce que** l'on prépare un mélange contenant
i) au moins un composé d'ammonium quaternaire a), comprenant éventuellement un monoalcool ramifié ou non ramifié contenant 1 à 4 atomes de carbone ;
ii) au moins un polyol contenant 5 à 12 atomes de carbone et
iii) éventuellement au moins un monoalcool non ramifié ou ramifié contenant 1 à 4 atomes de carbone.

17. Procédé de préparation d'une composition selon au moins l'une des revendications 1 à 15, **caractérisé en ce que** l'on alkyle
i) au moins une amine tertiaire NR₁R₂R₃, dans laquelle R₁ représente un groupe alkyle ou alcényle non ramifié ou ramifié contenant 12 à 36 atomes de carbone, un groupe R₅CONH(CH₂)ₙ- ou un groupe R₅COO(CH₂)ₙ-, dans lesquels R₅ représente un groupe alkyle ou alcényle contenant 12 à 36 atomes de carbone et n représente un nombre allant de 1 à 8 et R₂ et R₃ peuvent être, indépendamment l'un de l'autre, identiques ou différents et représentent -CH₃, CH₃CH₂-, CH₃CH₂CH₂-, CH₃CH₂CH₂CH₂- ou -CH₂CH₂(OH),
au moyen de
ii) au moins un agent alkylant, choisi parmi
a) R₄X, où R₄ représente -CH₃, CH₃CH₂-, CH₃CH₂CH₂-, CH₃CH₂CH₂CH₂- ou -CH₂CH(OH)CH₂(OH) et X représente Cl, I, Br, OSO₃H ou un méthosulfate et/ou
b) l'oxyde d'éthylène et un acide HX, dans lequel X représente Cl, I, Br, OSO₃H, un citrate ou un lactate,
en présence de
iii) au moins un polyol contenant 5 à 12 atomes de carbone et
iv) éventuellement au moins un monoalcool non ramifié ou ramifié contenant 1 à 4 atomes de carbone

18. Utilisation de compositions selon au moins l'une des revendications 1 à 15 pour la fabrication de produits cosmétiques, dermatologiques et pharmaceutiques, de préférence des produits de soins capillaires.

19. Utilisation selon la revendication 18, **caractérisée en ce que** les produits de soins capillaires sont des shampooings, des après-shampooings à rincer, des crèmes de rinçage, des lotions de rinçage, des soins capillaires, des produits de teinture et de coloration, des produits de permanente, des gels ou des produits coiffants sous forme d'aérosol, de pulvérisation et de liquide.
